Europäisches Patentamt

European Patent Office

Office européen des brevets

(11) Veröffentlichungsnummer: **0 033 863**
A1

(12) **EUROPÄISCHE PATENTANMELDUNG**

(21) Anmeldenummer: 81100367.2

(22) Anmeldetag: **19.01.81**

(51) Int. Cl.³: **C 07 C 49/443**, C 07 C 45/61, C 07 C 59/46

(30) Priorität: **08.02.80 DE 3004684**

(43) Veröffentlichungstag der Anmeldung: **19.08.81**
**Patentblatt 81/33**

(84) Benannte Vertragsstaaten: **CH DE FR GB IT LI**

(71) Anmelder: **Merck Patent Gesellschaft mit beschränkter Haftung, Frankfurter Strasse 250, D-6100 Darmstadt (DE)**

(72) Erfinder: **Habermehl, Gerhard, Prof. Dr., Jahnstrasse 19, D-6453 Seligenstadt (DE)**
Erfinder: **Hückmann, Karl-Heinz, Herrnstrasse 42, D-8711 Main-Bernheim (DE)**

(54) **Verfahren zur Herstellung von cis-Bicyclo(3.3.0)-octan-3,7-dion und seine Verwendung.**

(57) Die Anmeldung betrifft ein Verfahren zur Herstellung von cis-Bicyclo[3,3,0]octan-3,7-dion (I) durch Umsetzung von Glyoxal mit Diethyl-3-ketoglutarat, Di-n-propyl-3-ketoglutarat oder Diisopropyl-3-ketoglutarat in wäßriger, gepufferter Lösung zum entsprechenden 2,4,6,8-Tetracarbalkoxy-cis-bicyclo[3,3,0]octan-3,7-dion und Hydrolyse sowie Decarboxylierung dieser Verbindung.

I findet als Reagenz sowie als Zwischenprodukt bei der Herstellung von 9-Methano-prostacyclinen Verwendung.

EP 0 033 863 A1

Verfahren zur Herstellung von cis-Bicyclo/3.3.0/-
-octan-3,7-dion und seine Verwendung


Die Erfindung betrifft ein Verfahren zur Herstellung von
cis-Bicyclo/3.3.0/octan-3,7-dion (I).

I wird erstmals in Liebigs Ann. Chem. 426, 1-17 (1921)
beschrieben. Aus dieser Publikation sowie aus Chem. Tech.
8, 189-98 (1956) ist bereits ein Verfahren zur Herstellung
von I bekannt, bei dem man Trichlorethylidenmalonsäuredimethylester mit drei Molen Natriummalonsäuredimethylester zu einem Dehydro-1,1,2,2-Ethantetramalonsäuredi-
methylester umsetzt, aus diesem zwei Mole Kohlensäuredimethylester unter Bildung des Natriumsalzes des 2,4,6,8-
Tetracarbomethoxy-bicyclo/3.3.0/octen-3,7-dions abspaltet
und dieses Natriumsalz durch Reduktion und Decarboxylierung in I überführt.

Ferner wird in Tetrahedron Letters 47, 4885-4887, 1968
die Herstellung von I durch Umsetzen von Dimethyl-3-
ketoglutarat mit Glyoxal in wässeriger Lösung bei Raumtemperatur und nachfolgende Verseifung und Decarboxylierung des als Zwischenprodukt gebildeten 2,4,6,8-Tetra-
carbomethoxy-cis-bicyclo/3.3.0/octan-3,7-dion beschrieben.
Diese Reaktion von Glyoxal mit Dimethyl-3-ketoglutarat
verläuft jedoch nur mit einer Ausbeute von etwa 15 %,
und auch durch Änderung der Reaktionsbedingungen läßt
sich nur eine geringe Ausbeutesteigerung erreichen.

I wird jedoch als Reagenz und insbesondere als Zwischenprodukt bei Prostacyclin-Synthesen in steigendem Maße
benötigt, so daß Bedarf bestand an einem einfachen,
technisch befriedigenden Verfahren zur Herstellung
von I in guten Ausbeuten.

Aufgabe der Erfindung ist daher - ausgehend von dem bekannten Verfahren mittels Umsetzung von Glyoxal mit Dimethyl-3-ketoglutarat - ein Verfahren aufzufinden, nach dem cis-Bicyclo/3.3.0/octan-3,7-dion in gegenüber diesem bekannten Verfahren in wesentlich höheren Ausbeuten hergestellt werden kann. Diese Aufgabe wurde durch die vorliegende Erfindung gelöst.

Es wurde nämlich gefunden, daß überraschenderweise cis-Bicyclo/3.3.0/octan-3,7-dion in nahezu 90 %iger Ausbeute erhalten werden kann, wenn man Glyoxal mit Diethyl-3-ketoglutarat, Di-n-propyl-3-ketoglutarat oder Diisopropyl-3-ketoglutarat umsetzt. Dieses so vorteilhafte Ergebnis überrascht besonders, da keineswegs vorausgesehen werden konnte, daß gerade die Verwendung des Ethyl-, n-Propyl- oder Isopropylesters anstelle des Methylesters als Ausgangsprodukt die Ausbeute an dem für viele Synthesen wichtigen Zwischenprodukt cis-Bicyclo/3.3.0/octan-3,7-dion um ein Vielfaches steigern kann.

Gegenstand der Erfindung ist demgemäß ein Verfahren zur Herstellung von cis-Bicyclo/3.3.0/octan-3,7-dion durch Umsetzung von Glyoxal mit einem Dialkylester der 3-Ketoglutarsäure in wässeriger Lösung zum 2,4,6,8-Tetracarbalkoxy-cis-bicyclo/3.3.0/octan-3,7-dion und Hydrolyse sowie Decarboxylierung dieser Verbindung, das dadurch gekennzeichnet ist, daß man als Dialkylester der 3-Ketoglutarsäure Diethyl-3-ketoglutarat, Di-n-propyl-3-ketoglutarat oder Diisopropyl-3-ketoglutarat einsetzt.

0033863

Gegenstand der Erfindung ist ferner die Verwendung des erfindungsgemäß hergestellten cis-Bicyclo/3.3.0/octan-3,7-dions bei der Herstellung von 9-Methanoprostacyclinen.

Die als Ausgangsprodukt verwendeten Dialkylester der 3-Ketoglutarsäure sind zum Teil bekannt oder können analog den bekannten Verbindungen, beispielsweise durch Veresterung der 3-Ketoglutarsäure, hergestellt werden.

Die erfindungsgemäße Umsetzung von Glyoxal mit Diethyl-3-ketoglutarat, Di-n-propyl-3-ketoglutarat oder Diisopropyl-3-ketoglutarat wird in wässeriger, gepufferter Lösung durchgeführt. Der pH-Wert der Reaktionslösung liegt dabei im schwach sauren Bereich, das heißt zwischen pH 5 und etwa pH 7. Als besonders vorteilhaft hat sich ein pH-Wert von etwa 6 erwiesen. Die Einstellung des pH-Werts in der Ausgangsmischung der Reaktionsteilnehmer läßt sich auf beliebige bekannte Weise, zum Beispiel mittels geeigneter Puffer vornehmen, wobei besonders bevorzugt ein Citronensäure/$Na_2HPO_4$-Puffer ist. Man arbeitet in der Regel bei Temperaturen von 15 bis 30$^o$, besonders zweckmäßig bei Raumtemperatur. In besonderen Fällen können diese Temperaturen jedoch auch geringfügig über- bzw. unterschritten werden.

Diethyl-3-ketoglutarat, Di-n-propyl-3-ketoglutarat oder Diisopropyl-3-ketoglutarat und Glyoxal, das zweckmäßigerweise in wässeriger Lösung vorliegt, werden üblicherweise unter Rühren der wässerigen, gepufferten und auf den gewünschten pH-Wert eingestellten Lösung zugegeben. Zur Vollendung der Reaktion wird zweckmäßig längere Zeit, in der Regel 2 bis 5 Tage, gerührt. Das als Zwischenprodukt gebildete entsprechende 2,4,6,8-Tetracarbalkoxy-cis-bicyclo/3.3.0/octan-3,7-dion fällt kristallin aus und kann ohne weitere Reinigung in an sich bekannter zum gewünschten Endprodukt hydrolysiert und decarboxyliert werden.

Die Hydrolyse kann alkalisch oder vorzugsweise sauer vorgenommen werden. Man arbeitet in wässerigen Medien bei Temperaturen von in der Regel 50 bis 200°, vorzugsweise bei der Siedetemperatur des Reaktionsgemisches, wobei gleichzeitig die Decarboxylierung stattfindet. Als saure bzw. basische Katalysatoren eignen sich die üblicherweise bei der Hydrolyse von ß-Ketocarbonsäureestern verwendeten sauren bzw. basischen Verbindungen.

Bei Durchführung des erfindungsgemäßen Verfahrens erhält man außer dem gewünschten Reaktionsprodukt praktisch keine anderen Nebenprodukte, was dünnschichtchromatographisch gezeigt werden kann. Das cis-Bicyclo-/3.3.0/octan-3,7-dion wird aus der wässerigen neutralisierten Lösung durch Extraktion mit einem geeigneten Lösungsmittel, vorzugsweise Chloroform, abgetrennt.

Das Verfahrensprodukt wird als Reagenz und Feinchemikalie verwendet. Besondere Bedeutung hat cis-Bicyclo-/3.3.0/octan-3,7-dion als wertvolles Zwischenprodukt bei der Synthese von 9-Methanoprostacyclinen, wie beispielsweise in J. Chem. Soc., Chem. Commun. 1978, 1067 - 1068 beschrieben.

Die Erfindung wird durch folgende Beispiele erläutert:

Beispiel 1

a) 28,0 g Citronensäure-monohydrat (0,133 Mol) und 81,2 g $Na_2HPO_4 \cdot 2H_2O$ (0,456 Mol) werden in 3,4 l Wasser gelöst. Nach Überprüfung des pH-Wertes (pH 6) werden unter Rühren 65,4 g Diethyl-3-ketoglutarat (0,324 Mol) und 26,2 ml 30 %ige Glyoxallösung (0,162 Mol) zugegeben. Die Lösung wird noch fünf Tage weitergerührt. Das ausgefallene Kristallisat wird abgesaugt, mehrmals mit Wasser gewaschen und anschließend bis zur Gewichtskonstanz getrocknet. Man erhält 56,8 g 2,4,6,8-Tetracarbethoxy-cis-bi-cyclo/3.3.O/octan-3,7-dion (82 % d.Th.), das ohne weitere Reinigung weiterverarbeitet wird. Schmp. 107°.

Analyse: Schmp. 108° (nach Umkristallisation aus MeOH/DMSO (9:1))
IR(KBr): 3300, 3000, 2950, 1760, 1740, 1640 $cm^{-1}$;
$^1$H-NMR(CDCl$_3$): $\tau$=0,61(s), 8,7(t), 5,75(q);
MS (70eV): m/e = 426 (20%, M$^+$), 334 (100%).

b) Man gibt 40 g rohes 2,4,6,8-Tetracarbethoxy-cis-bicyclo/3.3.O/octan-3,7-dion (93,8 mMol) zu 270 ml Eisessig. Unter Rühren werden dann 68 ml Wasser und 10 ml $H_2SO_4$ konz. zugesetzt. Die Mischung wird bis zur klaren Lösung des organischen Materials am Rückfluß gekocht. Nach Zugabe von 400 ml Wasser, wobei keine Trübung der Lösung auftreten soll, wird noch 10 Stunden am Rückfluß gekocht, anschließend auf Raumtemperatur abgekühlt und mit $Na_2CO_3$ auf pH 7 eingestellt. Die wäßrige Lösung wird mit Chloroform extrahiert, die organische Phase anschließend unter vermindertem Druck eingeengt. Man erhält 12,8 g kristallines cis-Bicyclo[3.3.0]octan-3,7-dion (99 % der Theorie). Schmp. 84°. Nach Umkristallisation aus Ethylacetat/ Hexan (1:1) erhält man 11,6 g cis-Bicyclo[3.3.0]octan-3,7-dion (90 % der Theorie) in langen, farblosen Nadeln.
Schmp. 85°.
IR (KBr): 2970, 2910, 1735 cm$^{-1}$;
$^{1}$H-NMR(CDCl$_3$): $\tau$ = 6,95(m), 8,75(d), 9,0(d);
MS (70eV): m/e = 138 (36%, M$^{+}$).

Beispiel 2

680 ml Wasser werden durch Zugabe von 5,6 g Citronensäure-monohydrat und 16,3 g Na$_2$HPO$_4$·2H$_2$O auf pH 6 eingestellt. Unter Rühren gibt man 14,9 g Di-n-propyl-3-ketoglutarat und 5,2 ml 30 %ige Glyoxallösung zu. Die Reaktionslösung wird noch vier Tage weitergerührt. Man arbeitet wie in Beispiel 1a auf und erhält 12,6 g 2,4,6,8-Tetracarbopropyloxy-cis-bicyclo[3.3.0]octan-3,7-dion (81 % der Theorie), das ohne weitere Reinigung wie in Beispiel 1b hydrolysiert und decarboxyliert wird. Nach Umkristallisation des Reaktionsproduktes erhält man 3,3 g cis-Bicyclo[3.3.0]octan-3,7-dion (92 % der Theorie).
Schmp. 85°.

Beispiel 3

Aus 1,13 l Wasser, 9,3 g Citronensäure-monohydrat und 27,1 g Na$_2$HPO$_4$·2H$_2$O wird eine auf pH 6 gepufferte Lösung hergestellt. Unter Rühren gibt man 24,8 g Diisopropyl-3-ketoglutarat und 8,7 ml 30 %ige Glyoxallösung zu. Bei Raumtemperatur wird noch fünf Tage weitergerührt. Nach Aufarbeitung analog Beispiel 1a erhält man 21,6 g 2,4,6,8-Tetracarbisopropyloxy-cis-bicyclo[3.3.0]octan-3,7-dion (83 % der Theorie), das ohne weitere Reinigung analog der in Beispiel 1b beschriebenen Arbeitsweise hydrolysiert und decarboxyliert wird. Man erhält 5,6 g cis-Bicyclo$\lfloor$3.3.0$\rfloor$octan-3,7-dion (91 % der Theorie) nach Umkristallisation aus Ethylacetat/Hexan (1:1). Schmp. 85$^{\circ}$.

Merck Patent Gesellschaft
mit beschränkter Haftung
D a r m s t a d t

Patentansprüche:

1. Verfahren zur Herstellung von cis-Bicyclo/3.3.0/-
   octan-3,7-dion durch Umsetzung von Glyoxal mit
   einem Dialkylester der 3-Ketoglutarsäure in wässeriger Lösung zum 2,4,6,8-Tetracarbalkoxy-cis-
   bicyclo/3.3.0/octan-3,7-dion und Hydrolyse sowie
   Decarboxylierung dieser Verbindung, dadurch gekennzeichnet, daß man als Dialkylester der 3-Ketoglutarsäure Diethyl-3-ketoglutarat, Di-n-propyl-3-keto-
   glutarat oder Diisopropyl-3-ketoglutarat einsetzt.

2. Verfahren nach Anspruch 1, dadurch gekennzeichnet, daß
   die Umsetzung von Glyoxal mit Diethyl-3-ketoglutarat,
   Di-n-propyl-3-ketoglutarat oder Diisopropyl-3-keto-
   glutarat bei einem pH-Wert von 5 bis 7 durchgeführt
   wird.

3. Verwendung von nach Anspruch 1 oder 2 hergestelltem
   cis-Bicyclo/3.3.0/octan-3,7-dion bei der Herstellung
   von 9-Methano-prostacyclinen.

Europäisches Patentamt

**EUROPÄISCHER RECHERCHENBERICHT**

Nummer der Anmeldung

EP 81100367.2

| EINSCHLÄGIGE DOKUMENTE | | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | C 07 C 49/443 <br> C 07 C 45/61 <br> C 07 C 59/46 |
| | Keine Entgegenhaltungen | | |

**RECHERCHIERTE SACHGEBIETE (Int. Cl.³)**

C 07 C 49/00

C 07 C 45/00

---

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| WIEN | 06-04-1981 | REIF |

EPA form 1503.1   06.78